(19) Europäisches Patentamt
European Patent Office
Office européen des brevets

(11) Veröffentlichungsnummer: **0 287 690 B1**

# (12) EUROPÄISCHE PATENTSCHRIFT

(45) Veröffentlichungstag der Patentschrift: **02.09.92**

(51) Int. Cl.[5]: **C07C 217/18**, A61K 31/135

(21) Anmeldenummer: **87105803.8**

(22) Anmeldetag: **21.04.87**

## (54) Stabile Lösungsmitteladdukte von Z-1-(p-beta-Dimethylamino-ethoxyphenyl)-1-(p-hydroxyphenyl)-2-phenylbut-1-en.

(43) Veröffentlichungstag der Anmeldung:
**26.10.88 Patentblatt 88/43**

(45) Bekanntmachung des Hinweises auf die Patenterteilung:
**02.09.92 Patentblatt 92/36**

(84) Benannte Vertragsstaaten:
**AT BE CH DE ES FR GB GR IT LI LU NL SE**

(56) Entgegenhaltungen:
**EP-A- 0 002 097**
**EP-A- 0 107 208**
**GB-A- 1 099 093**

(73) Patentinhaber: **HEUMANN PHARMA GMBH & CO**
**Heideloffstrasse 18 - 28**
**W-8500 Nürnberg 1(DE)**

(72) Erfinder: **Grafe, Ingomar, Dr., Dipl.-Chem.**
**Auerbacher Strasse 18**
**W-8500 Nürnberg(DE)**
Erfinder: **Schickaneder, Helmut, Dr., Dipl.-Chem.**
**Moosäcker 25**
**W-8501 Eckental(DE)**
Erfinder: **Jungblut, Peter W., Prof. Dr., Dipl.-Biochem.**
**Konstanty-Gutschow-Strasse 8**
**W-3000 Hannover(DE)**
Erfinder: **Ahrens, Kurt Henning, Dr.**
**Praterstrasse 9**
**W-8500 Nürnberg(DE)**

(74) Vertreter: **Kraus, Walter, Dr. et al**
**Patentanwälte Kraus, Weisert & Partner**
**Thomas-Wimmer-Ring 15**
**W-8000 München 22(DE)**

EP 0 287 690 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue stereochemisch reine und stabile Addukte von Z-1-(p-β-Dimethylaminoethoxyphenyl)-1-(p-hydroxyphenyl)-2-phenylbut-1-en und bestimmten Lösungsmitteln, die aufgrund ihrer ausgeprägten antiestrogenen Aktivitäten bei der therapeutischen Behandlung von benignen Brusterkrankungen und hormonabhängigen Mammatumoren insbesondere durch percutane Applikation eingesetzt werden können.

Tamoxifen, d.h. 1-(p-β-Dimethylaminoethoxyphenyl)-1-phenyl-2-phenylbut-1-en, ist bereits aus einer Anzahl von Druckschriften, zum Beispiel aus der US-A-4 536 516, bekannt. Diese Druckschrift beschreibt auch die Behandlung von hormonabhängigen Tumoren mit Tamoxifen.

Aus der DE-OS 2 807 599 ist weiterhin bekannt, daß ein Metabolit des Tamoxifens, nämlich das Z-1-(p-β-Dimethylaminoethoxyphenyl)-1-(p-hydroxyphenyl)-2-phenylbut-1-en (Z-4-Hydroxytamoxifen), durch seine für die Rezeptorbildung wichtige Hydroxygruppe eine höhere antiestrogene Wirkung besitzt als das Grundmolekül.

Es hat sich jedoch gezeigt, daß für die antiestrogene Wirkung die stereochemische Reinheit des Moleküls entscheidend ist, da die E-Form des 4-Hydroxytamoxifens als Estrogen wirkt (vgl. zum Beispiel P.C. Rünitz u.a., J. Med. Chem. 25, 1056 (1982) und Biochem. Pharmacol. 32, 2941 (1983)).

Nach den Angaben der DE-OS 2 807 599 besitzt das Z-4-Hydroxytamoxifen zum Zeitpunkt seiner Herstellung einen Schmelzpunkt von 142 bis 144 ° C. Bereits nach einer Lagerzeit von einigen Wochen bei Raumtemperatur sinkt der Schmelzpunkt aber um ca. 4 ° C, was darauf zurückzuführen ist, daß der Gehalt des unerwünschten E-Isomeren ständig zunimmt. Diese Z-E-Isomerisierung von 4-Hydroxytamoxifen findet nicht nur in der Festsubstanz, sondern insbesondere auch in Lösung statt. Dabei isomerisiert das 4-Hydroxytamoxifen der Formel I-Z in das Derivat der Formel I-E gemäß folgendem Schema:

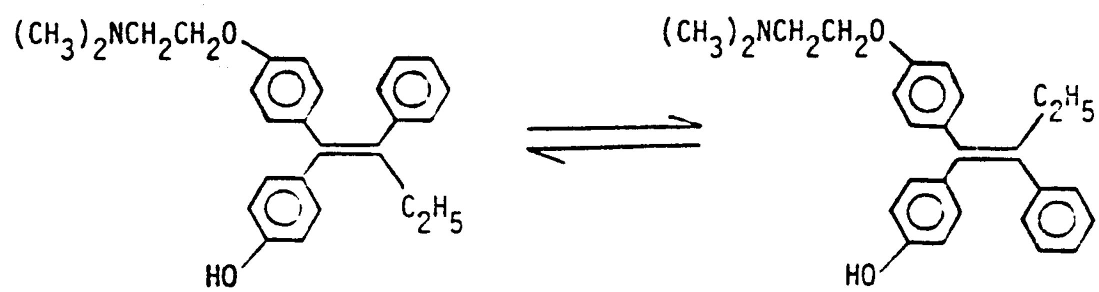

I-Z I-E

Das Gleichgewicht liegt bei einem Verhältnis von ca. 3:2 zugunsten der I-Z-Form.

Wie oben bereits ausgeführt, wirkt E-4-Hydroxytamoxifen (Formel I-E) als reines Estrogen, so daß - unabhängig von der Applikationsart -für die Behandlung von benignen Brusterkrankungen und für die Tumortherapie mit Z-4-Hydroxytamoxifen Präparate mit einem E-Isomerengehalt, der konstant unter 1% liegt, erforderlich sind.

Diese Forderung läßt sich mit den bisher bekannten, konformativ instabilen Formen des Z-4-Hydroxytamoxifens, wie sie in den oben erwähnten Druckschriften beschrieben werden, sowie mit dessen Hydrohalogeniden oder Alkalisalzen nicht erfüllen.

Der Erfindung liegt daher die Aufgabe zugrunde, stabile Formen von Z-4-Hydroxytamoxifen zur Verfügung zu stellen. Durch die Erfindung soll auch ein präparativ gangbares Verfahren zur Herstellung dieser Formen zur Verfügung gestellt werden, das auch die Herstellung größerer Mengen erlaubt.

Diese Aufgabe wird durch die Erfindung gelöst.

Gegenstand der Erfindung sind stabile Lösungsmitteladdukte von Z-1-(p-β-Dimethylaminoethoxyphenyl)-1-(p-hydroxyphenyl)-2-phenylbut-1-en (Z-4-Hydroxytamoxifen) der allgemeinen Formel I

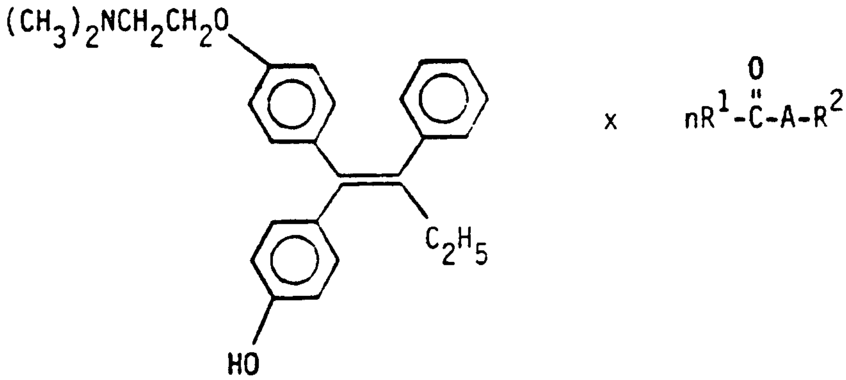

(I)

In der allgemeinen Formel I gibt die Teilformel

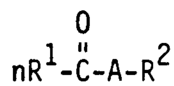

das Lösungsmittel an, das erfindungsgemäß das Addukt mit Z-4-Hydroxytamoxifen bildet. Darin steht $R^1$ für ein Wasserstoffatom, eine Phenyl-oder eine $C_1$-$C_6$-Alkylgruppe.

Als $C_1$-$C_6$-Alkylgruppen kommen geradkettige oder verzweigte $C_1$-$C_6$-Alkylgruppen in Betracht. Unter diesen werden $C_1$-$C_4$-Alkylgruppen, die geradkettig oder verzweigt, vorzugsweise geradkettig, sind, bevorzugt. Beispiele für solche $C_1$-$C_4$-Alkylgruppen sind die Methyl-, Ethyl-, n-Propyl-, i-Propyl-, n-Butyl-, i-Butyl-, sec.-Butyl- und tert.-Butylgruppe, wobei die Methyl- und die Ethylgruppe besonders bevorzugt werden. $R^2$ bedeutet eine geradkettige oder verzweigte $C_1$-$C_6$-Alkyl- oder -Alkenylgruppe, vorzugsweise eine geradkettige oder verzweigte $C_1$-$C_4$-Alkyl- oder -Alkenylgruppe, oder eine Acylgruppe mit bis zu 6 Kohlenstoffatomen. Beispiele für die $C_1$-$C_4$-Alkylgruppen sind die oben im Zusammenhang mit $R^1$ beschriebenen Gruppen. Beispiele für die $C_1$-$C_4$-Alkenylgruppen sind insbesondere die Vinylgruppe und die Butenylgruppe. Beispiele für Acylgruppen mit bis zu 6 Kohlenstoffatomen sind die Acetyl- und Propionylgruppe. Die Gruppen $R^1$ und $R^2$ können auch miteinander einen Kohlenwasserstoffring, beispielsweise einen Cyclohexylring, bilden. A bedeutet eine Einfachbindung, ein Sauerstoffatom, eine NH- oder eine Carbonylgruppe. n hat den wert 1/2 oder 1.

Einzelbeispiele für die durch die obige Teilformel angegebenen Lösungsmittel sind Aceton, Butanon, Acetylaceton, Mesityloxid, Essigsäureethylester und Acetessigsäureethylester, wobei Aceton, Acetylaceton und Mesityloxid bevorzugt werden.

Die erfindungsgemäßen Lösungsmitteladdukte bleiben bei Raumtemperatur und unter Streßbedingungen konformativ stabil. Deswegen sind sie für Arzneimittel und insbesondere für Arzneimittel, die für die percutane lokale Applikation vorgesehen sind, besonders geeignet.

Die erfindungsgemäßen Addukte werden durch ein Verfahren hergestellt, das eine entsprechende Isomerentrennung umfaßt. Bekannte Verfahren, wie sie beispielsweise in der DE-OS 2 835 536 und der EP-OS 2 097 beschrieben werden, sind nicht dazu geeignet, Produkte mit einem Gehalt an dem E-Isomeren von <1% herzustellen, da die Z-E-Trennung erst in der letzten Stufe durch wiederholte Kristallisation oder durch Säulenchromatographie erfolgt. Von R. McCagne wird zwar in J. Chem. Res. (S) 1986, 58 ein weiteres Verfahren beschrieben, das jedoch für die Herstellung größerer Mengen von Z-4-Hydroxytamoxifen ungeeignet ist, so daß es für praktische Zwecke nicht in Betracht kommt.

Überraschenderweise wurde nun erfindungsgemäß ein für größere Mengen präparativ gangbarer Weg zur Isomerentrennung, der auch keine kostspieligen Schutzgruppen erfordert, aufgefunden. Bei der Benzoylierung einer Verbindung der Formel II-Z und II-E

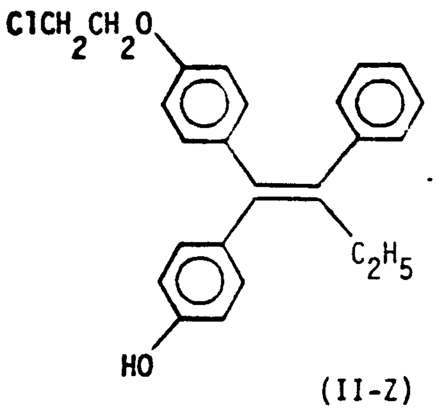

(II-Z)

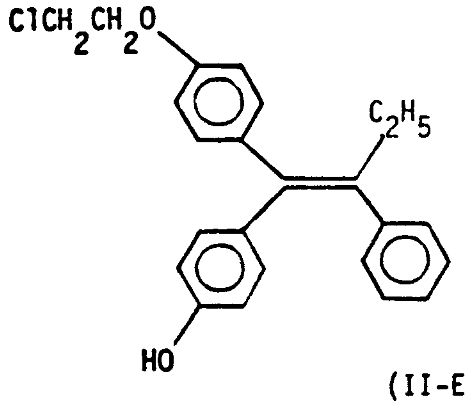

(II-E)

als Z-E-Gemisch, das nach an sich bekannten Verfahren hergestellt worden ist, wird nämlich ein Isomerengemisch erhalten, das als Feststoff anfällt, durch fraktionierte Kristallisation oder durch kinetisch kontrollierte Alkoholyse oder Aminolyse aufgetrennt werden kann, wobei das erhaltene E-Isomere in Z-4-Hydroxytamoxifen umgewandelt werden kann.

Ein weiterer Gegenstand der vorliegenden Erfindung ist daher ein Verfahren zur Herstellung stabiler Lösungsmitteladdukte von Z-1-(p-β-Dimethylaminoethoxyphenyl)-1-(p-hydroxyphenyl)-2-phenylbut-1-en (Z-4-Hydroxytamoxifen), das dadurch gekennzeichnet ist, daß man

a) ein Isomerengemisch der allgemeinen Formeln II-Z und II-E

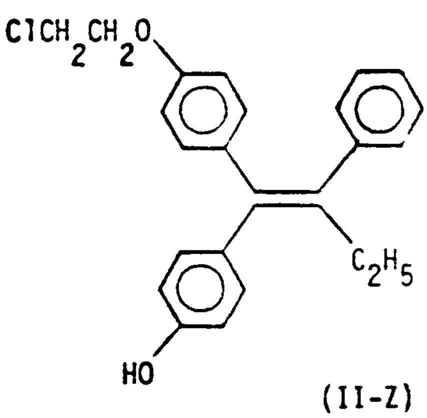

(II-Z)

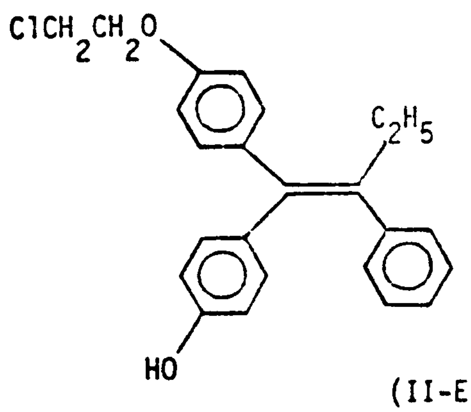

(II-E)

mit einem Säurechlorid der allgemeinen Formel III

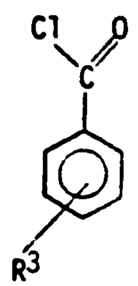

(III)

in der $R^3$ für ein Wasserstoffatom, eine $C_1$-$C_4$-Alkylgruppe, eine Arylgruppe, eine Nitrogruppe oder ein Halogenatom steht, zu einem Isomerengemisch der allgemeinen Formeln IV-Z und IV-E

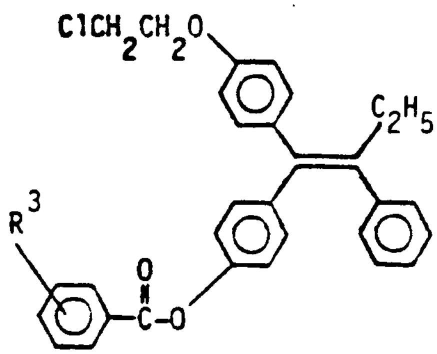

(IV-Z)

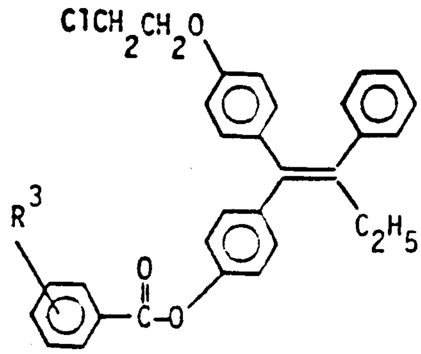

(IV-E)

umsetzt,

b) aus diesem Isomerengemisch durch fraktionierte Kristallisation der Verbindungen IV-E und IV-Z oder durch kinetisch kontrollierte Alkoholyse oder Aminolyse der Verbindung der allgemeinen Formel IV-Z das IV-E-Isomere als schwerer löslichen Anteil durch Filtration abtrennt,

c) die so erhaltene Verbindung der allgemeinen Formel IV-E mit Dimethylamin in die Verbindung der allgemeinen Formel V-Z

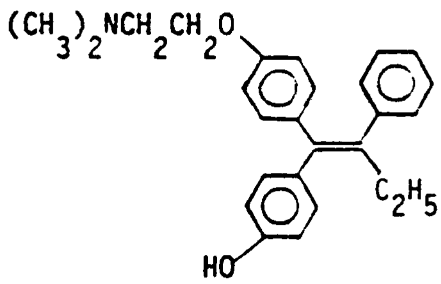

(V-Z)

umwandelt,

d) die Verbindung der allgemeinen Formel V-Z durch Kristallisation aus einem chlorierten Kohlenwasserstoff (CKW) in ein Addukt der allgemeinen Formel VI-Z

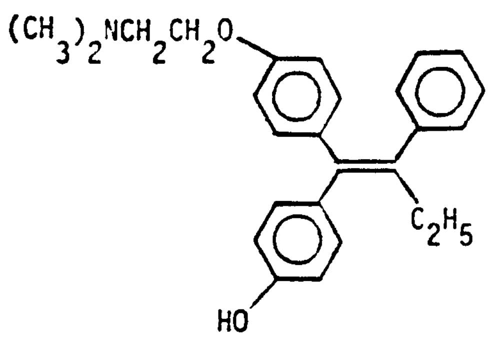

x yCKW

(VI-Z)

worin y den Wert 1/2 bis 1 hat, umwandelt

und daß man

e) die Verbindung der allgemeinen Formel VI-Z durch Kristallisation aus einer Carbonylverbindung der allgemeinen Formel VII

$$R^1-\overset{O}{\overset{\|}{C}}-A-R^2 \qquad \text{(VII)}$$

worin $R^1$, A und $R^2$ die oben angegebenen Bedeutungen besitzen, in das stereochemisch reine und stabile Lösungsmitteladdukt der allgemeinen Formel I überführt.

In der ersten Stufe des erfindungsgemäßen Verfahrens wird ein Isomerengemisch der allgemeinen Formeln II-Z und II-E

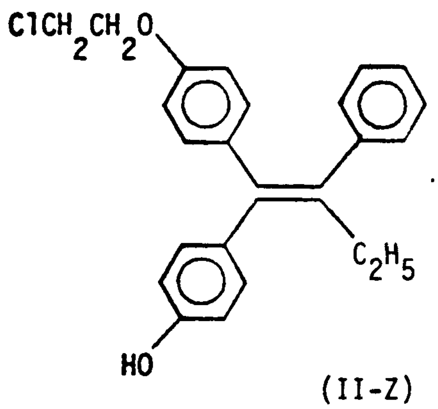
(II-Z)

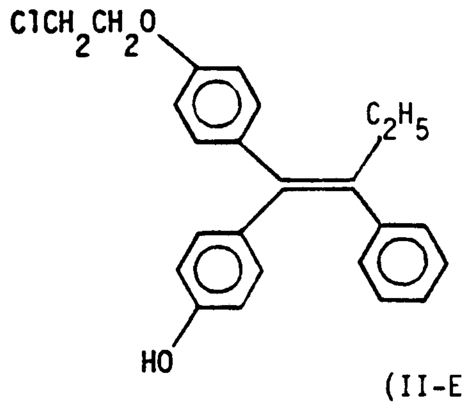
(II-E)

mit einem Säurechlorid der allgemeinen Formel III

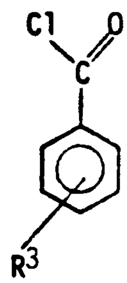
(III)

in der $R^3$ für ein Wasserstoffatom, eine $C_1$-$C_4$-Alkylgruppe, wie oben definiert, eine Arylgruppe, vorzugsweise eine Phenylgruppe, eine Nitrogruppe oder ein Halogenatom, beispielsweise ein Fluor-, Chlor-oder Bromatom, steht, zu einem Isomerengemisch der allgemeinen Formeln IV-Z und IV-E

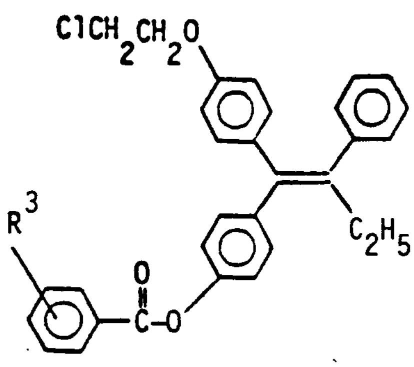
(IV-E)

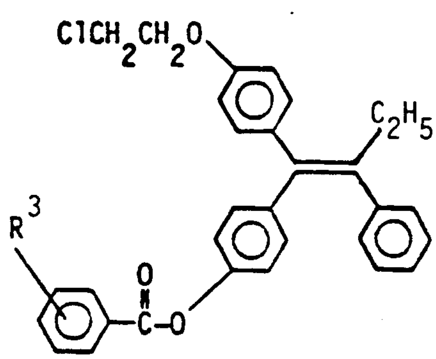
(IV-Z)

umgesetzt. In den allgemeinen Formeln IV-Z und IV-E hat $R^3$ die oben angegebene Bedeutung.

Das Isomerengemisch II-Z und II-E kann nach an sich bekannten Verfahren, wie sie beispielsweise von R. McCagne in J. Chem. Res. (S) 1986, 58 beschrieben werden, hergestellt werden.

Diese Stufe des erfindungsgemäßen Verfahrens, d.h. die Benzoylierung, wird in organischen Lösungsmitteln, beispielsweise aliphatischen oder aromatischen Kohlenwasserstoffen, zum Beispiel Hexan oder Toluol, durchgeführt. Das Reaktionsgemisch kann ein säureakzeptierendes Mittel, wie zum Beispiel Pottasche, und einen Phasentransferkatalysator, wie Tetrabutylammoniumhydrogenphosphat, enthalten.

Die Umsetzung erfolgt beispielsweise bei Temperaturen von -10 bis +25°C, vorzugsweise 0 bis +10°C. Das Molverhältnis von Isomerengemisch zu Säurechlorid der allgemeinen Formel III liegt im Bereich von 1,5:1 bis 1:1.

Die Aufarbeitung des Reaktionsgemisches geschieht in an sich bekannter Weise, zum Beispiel Abdampfen des Lösungsmittels und Kristallisation. Hierdurch wird das Isomerengemisch als Feststoff erhalten.

In der zweiten Stufe des erfindungsgemäßen Verfahrens wird das in der ersten Stufe erhaltene Isomerengemisch entweder konventionell durch fraktionierte Kristallisation der Verbindungen IV-E und IV-Z oder durch kinetisch kontrollierte Alkoholyse oder Aminolyse der Verbindung IV-Z aufgetrennt. Die fraktionierte Kristallisation erfolgt in an sich bekannter Weise. Die kinetisch kontrollierte Alkoholyse oder Aminolyse der Verbindung IV-Z kann beispielsweise unter Verwendung von Methanol, Ethanol oder Isopropanol als Alkohol bzw. von Piperidin, Pyrrolidin oder Diethylamin als Amin in einem Alkohol durchgeführt werden. Die Umsetzung kann bei Temperaturen im Bereich von 20°C bis zum Siedepunkt des jeweils verwendeten Alkohols durchgeführt werden. Bei der kinetisch kontrollierten Alkoholyse oder Aminolyse wird nur die Z-Form der Verbindung IV angegriffen, welche in die E-Form der Verbindung V umgewandelt wird. Diese fällt aus dem Lösungsmittel aus, während die IV-E-Verbindung darin gelöst bleibt.

Das E-Isomere der Verbindung V kann durch Filtration abgetrennt werden.

In der dritten Stufe des erfindungsgemäßen Verfahrens wird die Verbindung der allgemeinen Formel IV-E mit Dimethylamin in die Verbindung der allgemeinen Formel V-Z

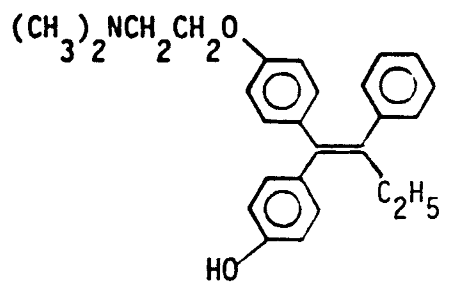

(V-Z)

umgewandelt.

Die Umsetzung kann beispielsweise in einem Wasser-Methylglykol-Gemisch durchgeführt werden. Sie wird bei einer Reaktionstemperatur von 20 bis 95°C, vorzugsweise 85 bis 95°C, durchgeführt. Das Molverhältnis der Verbindung IV-E zu Dimethylamin liegt im Bereich von 1:1,5 bis 1:1.

In der nächsten, d.h. der vierten, Stufe des erfindungsgemäßen Verfahrens wird die Verbindung der allgemeinen Formel V-Z durch Kristallisation aus einem chlorierten Kohlenwasserstoff (CKW) in ein Addukt der allgemeinen Formel VI-Z

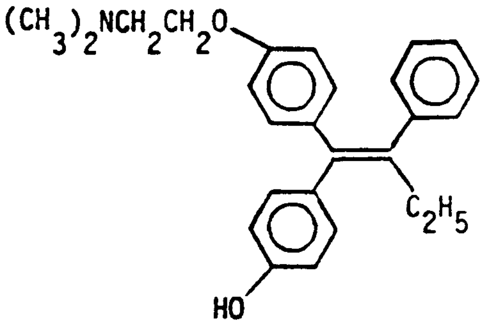

x y CKW (VI-Z)

worin y einen Wert von 1/2 bis 1 hat, umgewandelt. Als chlorierte Kohlenwasserstoffe kommen insbesondere Perchlorethylen, Trichlorethylen oder Chloroform in Betracht. Die Konzentration der Verbindung der allgemeinen Formel V-Z in dem chlorierten Kohlenwasserstoff beträgt zum Beispiel 1 bis 50 Gew.-%. Im einzelnen erfolgt die Kristallisation des Betains in an sich bekannter Weise durch Auflösen bei erhöhter Temperatur in dem chlorierten Kohlenwasserstoff und Abkühlenlassen.

Das erhaltene Addukt mit dem chlorierten Kohlenwasserstoff weist diskrete DSC-Maxima auf.

In der fünften, der letzten, Stufe des erfindungsgemäßen Verfahrens wird die Verbindung der allgemeinen Formel VI-Z durch Kristallisation aus einer Carbonylverbindung der allgemeinen Formel VII

$$R^1-\overset{O}{\overset{\|}{C}}-A-R^2 \qquad (VII)$$

worin $R^1$, $R^2$ und A wie oben definiert sind, in das erfindungsgemäße stabile Lösungsmitteladdukt der allgemeinen Formel I überführt. Dabei kann das Lösungsmittel der allgemeinen Formel VII auch im Gemisch mit einem inerten unpolaren Lösungsmittel, beispielsweise Hexan, verwendet werden. Die Konzentration der Verbindung der allgemeinen Formel VI-Z in dem Lösungsmittel beträgt beispielsweise 1 bis 50 Gew.-%.

Diese Stufe des erfindungsgemäßen Verfahrens wird wie folgt durchgeführt: Auflösen der Verbindung der allgemeinen Formel VI-Z bei erhöhter Temperatur in der Carbonylverbindung bzw. dem Gemisch aus Carbonylverbindung und inertem unpolaren Lösungsmittel bei erhöhter Temperatur, beispielsweise bis zum Siedepunkt des verwendeten Lösungsmittels, und Abkühlenlassen. Das sich beim Abkühlen abscheidende erfindungsgemäße Lösungsmitteladdukt kann in einfacher Weise durch Filtration abgetrennt werden.

Definierte Maxima sowie die spezifischen IR-Absorptionsbanden der Carbonylfrequenzen beweisen, daß es sich bei den erfindungsgemäßen Produkten um stabile Lösungsmitteladdukte handelt. In der nachstehenden Tabelle sind die DSC-Maxima der erfindungsgemäßen Lösungsmitteladdukte des Z-4-Hydroxytamoxifens zusammengestellt.

| Carbonylverbindung der allgemeinen Formel V | DSC-Maxima (°C) der Addukte (Heizrate 2°C/min) | IR (KBr) ν-C=O ($cm^{-1}$) |
|---|---|---|
| Aceton | 78, 138 | 1711 |
| Butanon | 71, 91, 146 | 1716 |
| Acetylaceton | 123, 133 | 1612, 1516 |
| Mesityloxid | 111, 139, 159 | 1687, 1610 |
| Essigsäureethylester | 92, 144, 148 | 1737 |
| Acetessigsäureethylester | 92, 130, 158 | 1735, 1715, 1650, 1630 |

DTA-Gerät: Mettler FP 85

IR-Gerät: Perkin-Elmer, Modell 1420

Aus den deutlich verschobenen Carbonylfrequenzen der Lösungsmitteladduktmoleküle gegenüber den freien Carbonylverbindungen (Formel V) erkennt man, daß die Lösungsmittelmoleküle sehr fest im Kristall gebunden sind.

Wie oben bereits ausgeführt, sind die erfindungsgemäßen Lösungsmitteladdukte aufgrund ihrer konformativen Stabilität besonders gut zur Herstellung von Arzneimitteln geeignet. Insbesondere sind sie für die Herstellung von Arzneimittel für die percutane lokale Applikation geeignet.

Ein weiterer Gegenstand der Erfindung sind daher Arzneimittel,die als Wirkstoff mindestens ein stabiles Lösungsmitteladdukt, wie oben beschrieben, sowie übliche Träger- und Hilfsstoffe enthalten.

Bei der Herstellung von Arzneimitteln für die percutane Applikation sind als pharmazeutische Hilfs- und Trägerstoffe besonders geeignet: mit Wasser mischbare Lösungsmittel, wie Ethanol, Isopropylalkohol, Propylenglykol, Dimethylsulfoxid, Dimethylformamid, Tetrahydrofuran, einzeln oder in Mischung mit jeweils vorzugsweise 50% Wasser. Derartige Lösungen können direkt aufgetragen oder zu Salben oder Cremes verarbeitet in geeigneter Dosierung eingesetzt werden. Für Salben oder Cremes geeignete Hilfsstoffe, in denen der Wirkstoff entweder gelöst oder suspendiert vorliegt, sind Fettsäureester, wie Ethylstearat, Isopropylmyristat, Isopropylpalmitat, Ölsäureoleylester, Capryl/Caprinsäureester von gesättigten Fettalkoholen der Kettenlänge $C_8$ bis $C_{12}$ u.a.; Triglyceride, wie Capryl/Caprinsäuretriglycerid, Triglyceridgemische mit Pflanzenfettsäuren der Kettenlänge $C_8$ bis $C_{12}$ u.a.; Fettalkohole, wie Cetylstearylalkohol, Oleylalkohol; Fettsäuren, wie Ölsäure und Stearinsäure, Tenside, zum Beispiel nichtionogene, wie polyoxyethyliertes Sorbitan-Monooleat, Glycerinmonostearat, Polyoxyethylenstearat; kationaktive, wie Cetyltrimethylammoniumchlorid, anionaktive, wie Natriumlaurylsulfat und Fettalkoholethersulfat; ampholytische, wie Lecithin, Stabilisatoren, wie hochdisperses Siliciumdioxid, Montmorillonite, Antioxidantien, wie Tocopherole, Butylhydroxyanisol.

Die erfindungsgemäßen Arzneimittel enthalten pro Dosiseinheit etwa $10^{-8}$ g bis etwa 0,2 g Lösungsmitteladdukt von Z-4-Hydroxytamoxifen. Gehalte von $10^{-6}$ g bis 0,1 g werden bevorzugt. Eine Dosiseinheit des erfindungsgemäßen Arzneimittels enthält $10^{-8}$ g bis 0,2 g, vorzugsweise 0,01 g bis 0,2 g, des erfindungsgemäßen Lösungsmitteladdukts. Die Dosiseinheit kann ein- bis mehrmals täglich verabreicht werden.

Im Einzelfall, und zwar insbesondere in Abhängigkeit vom individuellen Verhalten gegenüber dem erfindungsgemäß vorgeschlagenen Wirkstoff, dem Zustand der Patientin und ähnlicher Faktoren, kann es gegebenenfalls erforderlich sein, von den genannten Mengen abzuweichen. So gibt es zum Beispiel Fälle, wo mit weniger als der oben genannten Mindestmenge ausgekommen werden kann, während in anderen Fällen die genannte obere Grenze überschritten werden muß.

Die Erfindung wird in den Beispielen erläutert.

**Beispiel 1**

Herstellung eines Komplexes von Z-4-Hydroxytamoxifen mit einem chlorierten Kohlenwasserstoff

a) Darstellung von Z-E-1-(4-Benzoyloxyphenyl)-1-[(2-chlorethoxy)phenyl]-2-phenylbuten(1)

Eine salzsaure Lösung von 1-[4-(2-Chlorethoxy)phenyl]-1-(4-hydroxyphenyl)-2-phenylbuten, hergestellt nach R. McCagne, J. Chem. Res. (S) 1986, 58 bei 0,5 Mol Ansatzgröße, wird im Vakuum eingeengt und einer Wasserdampfdestillation unterwofen, bis keine organischen Verbindungen mehr übergehen.

Man nimmt die organische Verbindung in 300 ml Toluol auf, führt eine Phasentrennung durch und gibt die organische Phase zu einem Gemisch aus 0,6 Mol Pottasche in 250 ml Wasser und 3 ml 50%igem Tetrabutylammoniumhydrogenphosphat.

Bei maximal 40 ° C tropft man in einer halben Stunde unter Rühren 0,75 Mol Benzoylchlorid zu und rührt bei 20 bis 30 ° C, bis kein $CO_2$ mehr entweicht. Nach Phasentrennung wird das Lösungsmittel abgezogen und der Rückstand (260 g) in 260 ml n-Butanol aufgenommen.

Diese Lösung tropft man in ca. 8 bis 16 Stunden bei 0 bis 10 ° C in 1 Liter Methanol, so daß sich, bedingt durch die langsame Kristallisation, keine Klumpen bilden. Man läßt das Gemisch noch mindestens 24 Stunden bei 0 ° C stehen, saugt dann ab und wäscht mit Methanol nach. Man kann bei 50 ° C im Vakuum trocknen.

Ausbeute: 122 g (Z-E-Gemisch) = 51% d.Th.

DSC max = 107 + 109 ° C (2 ° C/min)

$^1$H-NMR-Daten ($CDCl_3$): 0,95 t (3H), 2,5 q (2H), 3,7 m (2H), 4,1 t (1 H), 4,2 t (1H), 6,6 - 8,3 m (18 H) ppm

b) Z-E-Trennung der Olefine aus a)

0,2 Mol des Z-E-Gemisches aus a) werden in 500 ml Methanol mit 0,1 Mol Piperidin 3 Stunden unter Rückfluß gekocht und 50 ° C abgesaugt. Man wäscht mit 100 ml Methanol nach.

Ausbeute: 14 g E-1-(4-Benzoyloxyphenyl)-1-[4-(2-chlorethoxy)phenyl]-2-phenylbuten(1)

Gehalt >90%

DSC max = 133 ° C (2 ° C/min)

$^1$H-NMR-Daten ($CDCl_3$): 0,95 t (3H), 2,5 q (2H), 3,8 t (2H), 4,25 t (2H), 7 - 8,3 m (18 H) ppm

$c_1$) Darstellung von Z-4-Hydroxytamoxifen-Chloroform-Komplex

0,3 Mol E-Isomeres der Benzoylverbindung aus b) werden in 0,3 Liter Glykol, 0,2 Liter Methylglykol und 50 ml 40%igem Dimethylamin bei 85 ° C gelöst. In 3 Stunden tropft man 100 ml 40%iges Dimethylamin zu, wobei kaum Amin entweicht. Dann heizt man auf 95 ° C und vertreibt das überschüssige Amin mit einem Stickstoffstrom. Nach Zugabe von 200 ml Wasser stellt man einen pH-Wert von ca. 3 mit 30%iger Schwefelsäure ein und extrahiert das Gemisch bei 30 ° C mit einem Gemisch aus 150 ml Toluol und 50 ml Spezialbenzin. Nach Zugabe von 50 g Kochsalz extrahiert man die wäßrige Phase zweimal mit je 200 ml Methylenchlorid und wäscht die vereinigten organischen Phasen mit 5%iger Kochsalzlösung und mit verdünntem Ammoniak. Das Lösungsmittel wird abgezogen und der Rückstand (170 g) aus 0,5 l Chloroform kristallisiert. Nach 24 Stunden saugt man ab.

Ausbeute: 106 g

$C_{27}H_{30}Cl_3NO_2$ (FG 506,88) = 69% d.Th.

DSC max = 104 - 138 ° C (2 ° C/min)

$c_2$) Darstellung von Z-4-Hydroxytamoxifen-Perchlorethylen-Komplex

Man verfährt wie in $c_1$), jedoch erfolgt die Kristallisation aus Perchlorethylen.

Ausbeute: 117 g

$C_{28}H_{29}Cl_4NO_2$ (FG 553,33) = 71% d.Th.

DSC max = 135 ° C (2 ° C/min)

**Beispiel 2**

Herstellung des Z-4-Hydroxytamoxifen-Aceton-Komplexes

0,1 Mol des Chloroform- oder Perchlorethylenkomplexes des Z-4-Hydroxytamoxifens aus den Beispielen 1$c_1$) und 1$c_2$) werden aus 300 ml Aceton umkristallisiert und bei 10 ° C abgesaugt.

Ausbeute: 36 g

$C_{29}H_{35}NO_3$ (FG 445,58) = 81% d.Th.

**Beispiel 3**

Herstellung des Z-4-Hydroxytamoxifen-Mesityloxid-Komplexes

0,1 Mol der 4-Hydroxytamoxifenkomplexe aus den Beispielen 1c2) und 2 werden in 400 ml Mesityloxid von 60 ° C eingetragen. Wenn die Substanz gelöst ist, kühlt man auf 15 ° C, saugt ab und wäscht mit 75 ml Methyltert.-butylether nach.
Ausbeute: 34,5 g = 79% d.Th.
Zusammensetzung: 2 x $C_{26}H_{29}NO_2$ x $C_6H_{10}O$

| | | | | |
|---|---|---|---|---|
| Analyse: | berechnet:<br>gefunden: | C 79,77;<br>C 80,00; | H 7,86;<br>H 7,79; | N 3,21<br>N 3,22 |

$^1$H-NMR-Daten ($CDCl_3$): 0,9 t (6H), 1,9 s (3H), 2,05 s (6H) 4 J 1 Hz, 2,15 s (12 H), 2,5 m (8H), 3,9 t (4H), 6,15 s breit (1H), 6,5 - 7,3 m (26 H) ppm

Strukturisomeres E-4-Hydroxytamoxifen ist nicht erkennbar.

## Patentansprüche

**Patentansprüche für folgende Vertragsstaaten : AT, BE, CH, DE, FR, GB, IT, LI, LU, NL, SE**

1. Stabile Lösungsmitteladdukte von Z-1-(p-β-Dimethylaminoethoxyphenyl)-1-(p-hydroxyphenyl)-2-phenylbut-1-en der allgemeinen Formel I

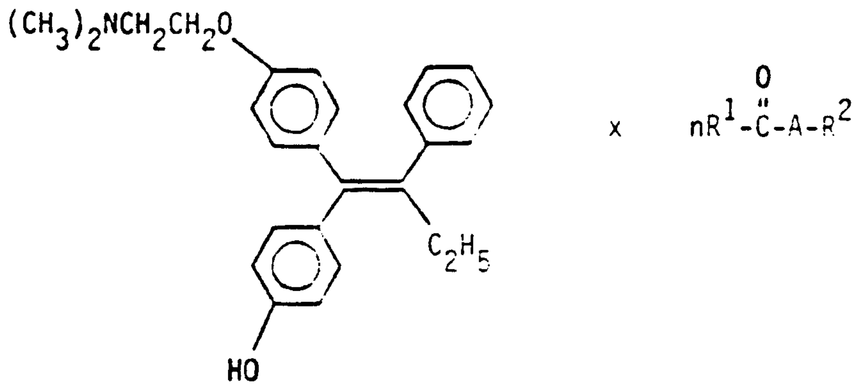

(I)

in der $R^1$ für ein Wasserstoffatom, eine Phenylgruppe oder eine $C_1$-$C_6$-Alkylgruppe steht, $R^2$ für eine geradkettige oder verzweigte $C_1$-$C_6$-Alkylgruppe, eine geradkettige oder verzweigte Alkenylgruppe oder eine Acylgruppe mit bis zu 6 Kohlenstoffatomen steht oder $R^1$ und $R^2$ miteinander einen Ring bilden, A eine Einfachbindung, ein Sauerstoffatom, eine NH- oder eine Carbonylgruppe bedeutet und n den Wert 1/2 oder 1 hat.

2. Addukte nach Anspruch 1, dadurch **gekennzeichnet,** daß $R^1$ und $R^2$ jeweils eine Methylgruppe bedeuten, daß A für eine Einfachbindung steht und daß n den Wert 1 hat.

3. Addukte nach Anspruch 1, dadurch **gekennzeichnet,** daß $R^1$ für eine Methylgruppe steht, $R^2$ die Gruppierung $-CH=C(CH_3)_2$ bedeutet, A für eine Einfachbindung steht und n den Wert 1/2 hat.

4. Addukte nach Anspruch 1, dadurch **gekennzeichnet,** daß das Lösungsmittel Aceton, Acetylaceton oder Mesityloxid ist.

5. Verfahren zur Herstellung von stabilen Lösungsmitteladdukten von Z-1-(p-β-Dimethylaminoethoxyphenyl)-1-(p-hydroxyphenyl)-2-phenylbut-1-en der allgemeinen Formel I

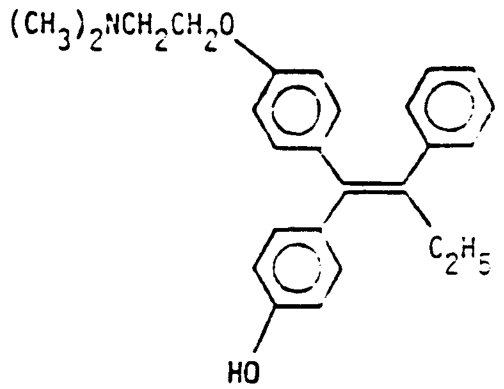

x

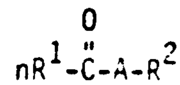

(I)

in der $R^1$ für ein Wasserstoffatom, eine Arylgruppe oder eine $C_1$-$C_6$-Alkylgruppe steht, $R^2$ für eine geradkettige oder verzweigte $C_1$-$C_6$-Alkylgruppe, eine geradkettige oder verzweigte Alkenylgruppe oder eine Acylgruppe mit bis zu 6 Kohlenstoffatomen steht oder $R^1$ und $R^2$ miteinander einen Ring bilden, A eine Einfachbindung, ein Sauerstoffatom, eine NH- oder eine Carbonylgruppe bedeutet und n den Wert 1/2 oder 1 hat, dadurch **gekennzeichnet,** daß man

a) ein Isomerengemisch der allgemeinen Formeln II-Z und II-E

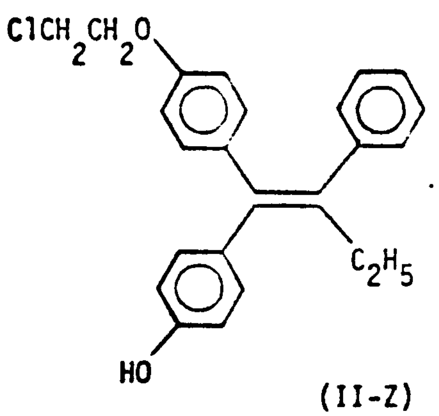

(II-Z)

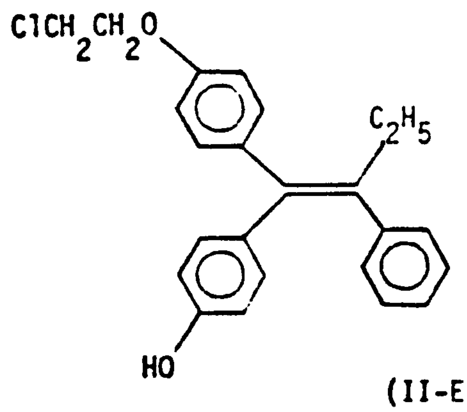

(II-E)

mit einem Säurechlorid der allgemeinen Formel III

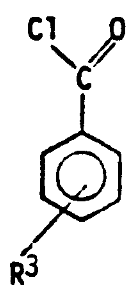

(III)

in der $R^3$ für ein Wasserstoffatom, eine $C_1$-$C_4$-Alkylgruppe, eine Arylgruppe, eine Nitrogruppe oder ein Halogenatom steht, zu einem Isomerengemisch der allgemeinen Formeln IV-Z und IV-E

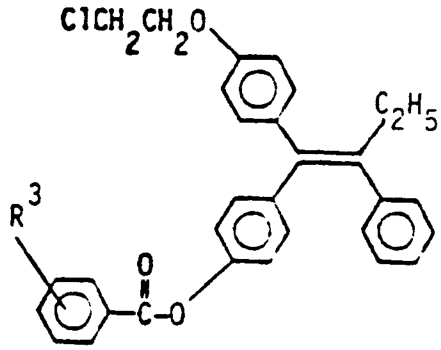

(IV-Z)

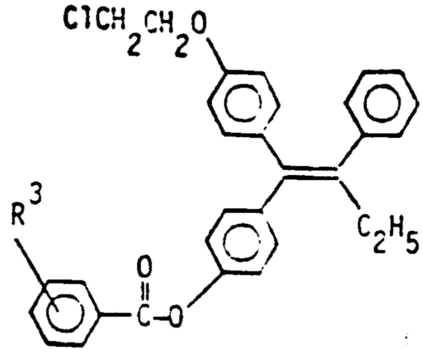

(IV-E)

umsetzt,

b) aus diesem Isomerengemisch durch fraktionierte Kristallisation der Verbindungen IV-E und IV-Z oder durch kinetisch kontrollierte Alkoholyse oder Aminolyse der Verbindung der allgemeinen Formel IV-Z das IV-E-Isomere als schwerer löslichen Anteil durch Filtration abtrennt,

c) die so erhaltene Verbindung der allgemeinen Formel IV-E mit Dimethylamin in die Verbindung der allgemeinen Formel V-Z

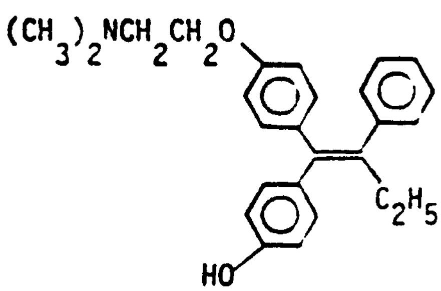

(V-Z)

umwandelt,

d) die Verbindung der allgemeinen Formel V-Z durch Kristallisation aus einem chlorierten Kohlenwasserstoff (CKW) in ein Addukt der allgemeinen Formel VI-Z

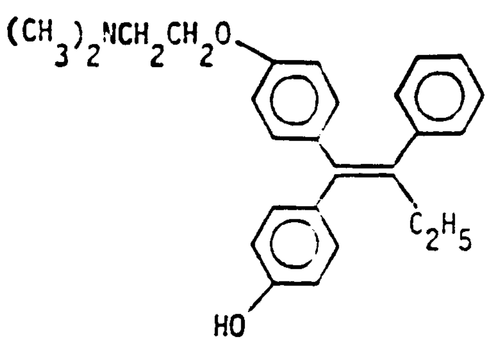

x yCKW

(VI-Z)

worin y den Wert 1/2 bis 1 hat, umwandelt

und daß man

e) die Verbindung der allgemeinen Formel VI-Z durch Kristallisation aus einer Carbonylverbindung der allgemeinen Formel VII

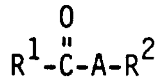

(VII)

worin $R^1$, A und $R^2$ die oben angegebenen Bedeutungen besitzen, in das stereochemisch reine und stabile Lösungsmitteladdukt der allgemeinen Formel I überführt.

6. Arzneimittel, dadurch **gekennzeichnet,** daß es ein stabiles Lösungsmitteladdukt nach Anspruch 1 sowie übliche Träger- und Hilfsstoffe enthält.

7. Verwendung von stabilen Lösungsmitteladdukten von Z-1-(p-β-Dimethylaminoethoxyphenyl)-1-(p-hydroxyphenyl)-2-phenylbut-1-en der allgemeinen Formel I

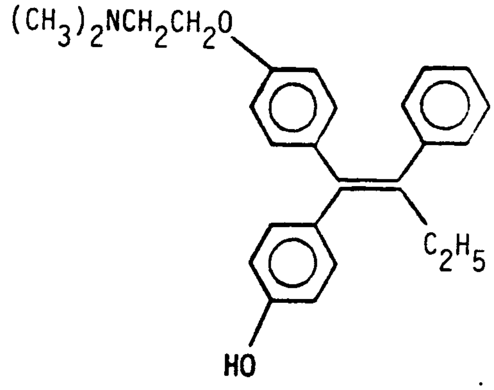

x

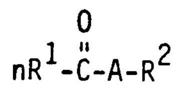

( I )

in der $R^1$ für ein Wasserstoffatom, eine Phenylgruppe oder eine $C_1$-$C_6$-Alkylgruppe steht, $R^2$ für eine geradkettige oder verzweigte $C_1$-$C_6$-Alkylgruppe, eine geradkettige oder verzweigte Alkenylgruppe oder eine Acylgruppe mit bis zu 6 Kohlenstoffatomen steht oder $R^1$ und $R^2$ miteinander einen Ring bilden, A eine Einfachbindung, ein Sauerstoffatom, eine NH- oder eine Carbonylgruppe bedeutet und n den Wert 1/2 oder 1 hat, zur Herstellung eines Arzneimittels für die percutane Behandlung von benignen und malignen Tumoren.

**Patentanspruch für folgende Vertragsstaaten : ES, GR**

1. Verfahren zur Herstellung von stabilen Lösungsmitteladdukten von Z-1-(p-β-Dimethylaminoethoxyphenyl)-1-(p-hydroxyphenyl)-2-phenylbut-1-en der allgemeinen Formel I

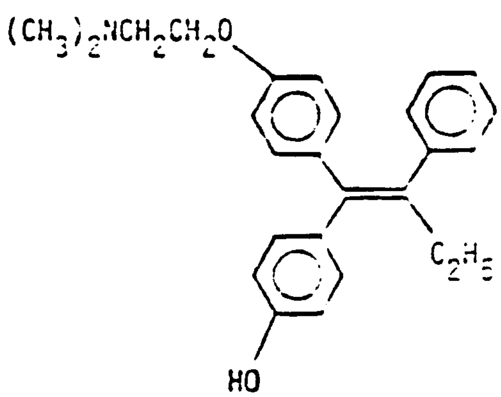

x

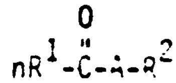

(I)

in der $R^1$ für ein Wasserstoffatom, eine Arylgruppe oder eine $C_1$-$C_6$-Alkylgruppe steht, $R^2$ für eine

geradkettige oder verzweigte $C_1$-$C_6$-Alkylgruppe, eine geradkettige oder verzweigte Alkenylgruppe oder eine Acylgruppe mit bis zu 6 Kohlenstoffatomen steht oder $R^1$ und $R^2$ miteinander einen Ring bilden, A eine Einfachbindung, ein Sauerstoffatom, eine NH- oder eine Carbonylgruppe bedeutet und n den Wert 1/2 oder 1 hat, dadurch **gekennzeichnet,** daß man

a) ein Isomerengemisch der allgemeinen Formeln II-Z und II-E

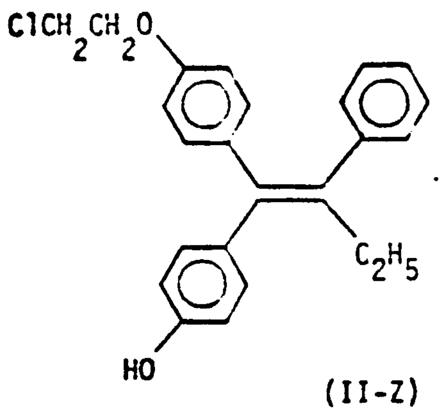
(II-Z)

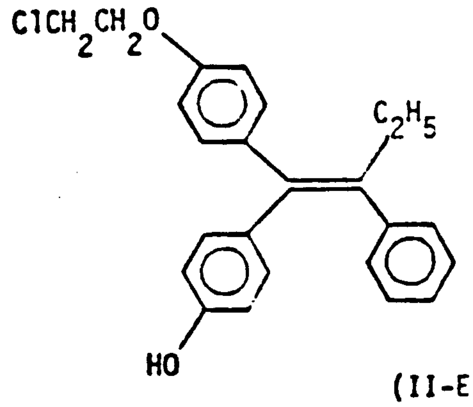
(II-E)

mit einem Säurechlorid der allgemeinen Formel III

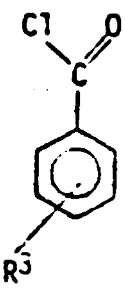
(III)

in der $R^3$ für ein Wasserstoffatom, eine $C_1$-$C_4$-Alkylgruppe, eine Arylgruppe, eine Nitrogruppe oder ein Halogenatom steht, zu einem Isomerengemisch der allgemeinen Formeln IV-Z und IV-E

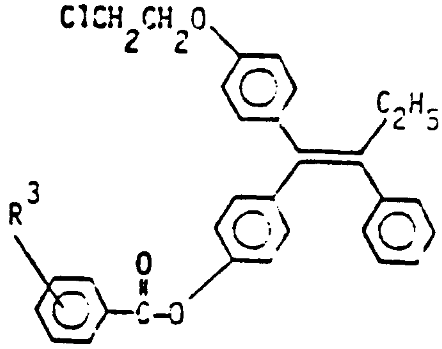
(IV-Z)

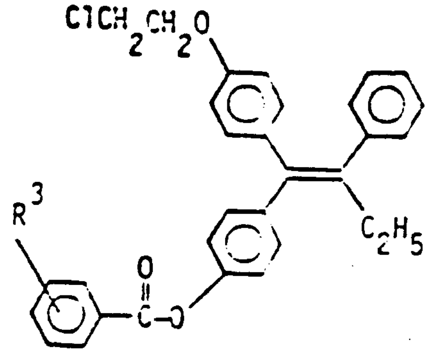
(IV-E)

umsetzt,

b) aus diesem Isomerengemisch durch fraktionierte Kristallisation der Verbindungen IV-E und IV-Z oder durch kinetisch kontrollierte Alkoholyse oder Aminolyse der Verbindung der allgemeinen

Formel IV-Z das IV-E-Isomere als schwerer löslichen Anteil durch Filtration abtrennt,
c) die so erhaltene Verbindung der allgemeinen Formel IV-E mit Dimethylamin in die Verbindung der allgemeinen Formel V-Z

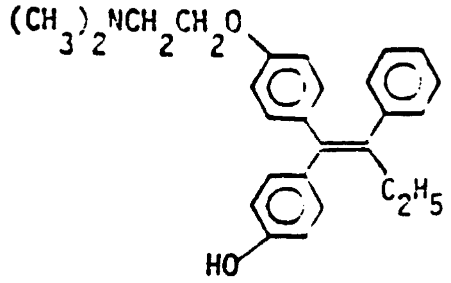

(V-Z)

umwandelt,
d) die Verbindung der allgemeinen Formel V-Z durch Kristallisation aus einem chlorierten Kohlenwasserstoff (CKW) in ein Addukt der allgemeinen Formel VI-Z

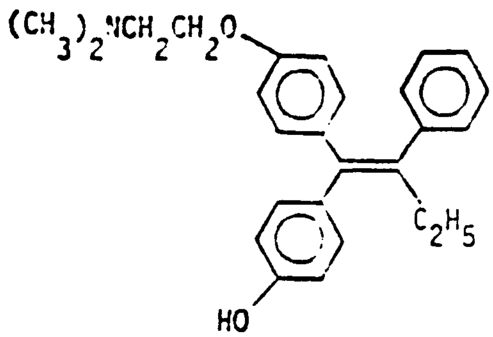

x yCKW (VI-Z)

worin y den Wert 1/2 bis 1 hat, umwandelt
und daß man
e) die Verbindung der allgemeinen Formel VI-Z durch Kristallisation aus einer Carbonylverbindung der allgemeinen Formel VII

$$R^1-\overset{O}{\overset{\|}{C}}-A-R^2 \qquad (VII)$$

worin $R^1$, A und $R^2$ die oben angegebenen Bedeutungen besitzen, in das stereochemisch reine und stabile Lösungsmitteladdukt der allgemeinen Formel I überführt.

**Claims**
**Claims for the following Contracting States : AT, BE, CH, DE, FR, GB, IT, LI, LU, NL, SE**

**1.** Stable solvent adducts of Z-1-(p-β-dimethylaminoethoxyphenyl)-1-(p-hydroxyphenyl)-2-phenylbut-1-ene corresponding to the general formula I

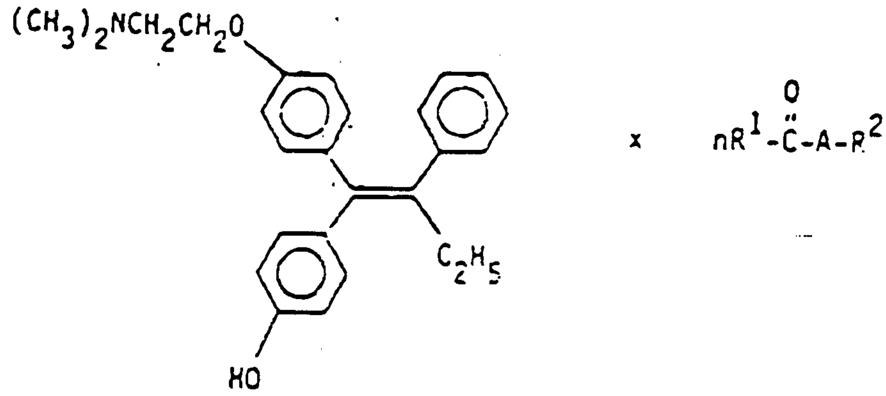

(I)

in which $R^1$ stands for a hydrogen atom, a phenyl group or a $C_1$-$C_6$ alkyl group, $R^2$ stands for a straight chain or branched $C_1$-$C_6$ alkyl group, a straight chain or branched alkenyl group or an acyl group having up to 6 carbon atoms or $R^1$ and $R^2$ together form a ring, A denotes a single bond, an oxygen atom, an NH group or a carbonyl group and n has the value 1/2 or 1.

**2.** Adducts according to claim 1, characterised in that $R^1$ and $R^2$ denote each a methyl group, A stands for a single bond and n has the value 1.

**3.** Adducts according to claim 1, characterised in that $R^1$ stands for a methyl group, $R^2$ stands for the group-$CH=C(CH_3)_2$, A stands for a single bond and n has the value 1/2.

**4.** Adducts according to claim 1, characterised in that the solvent is acetone, acetyl acetone or mesityl oxide.

**5.** A process for the preparation of stable solvent adducts of Z-1-(p-β-dimethylaminoethoxyphenyl)-1-(p-hydroxyphenyl)-2-phenylbut-1-ene corresponding to the general formula I

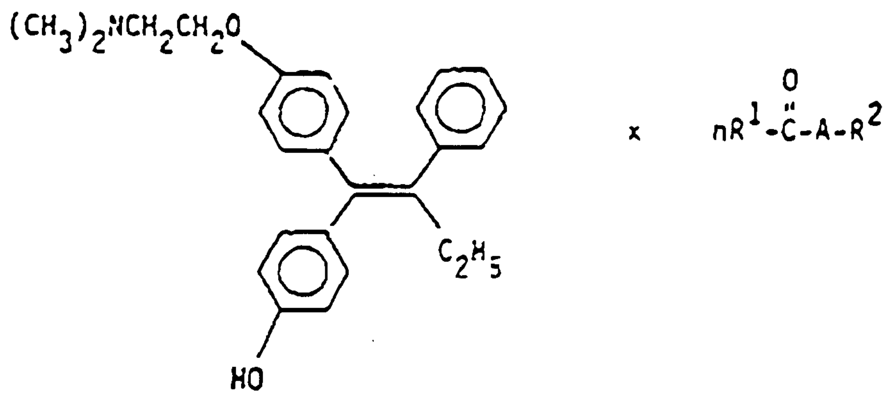

(I)

in which $R^1$ stands for a hydrogen atom, an aryl group or a $C_1$-$C_6$ alkyl group, $R^2$ stands for a straight chain or branched $C_1$-$C_6$ alkyl group, a straight chain or branched alkenyl group or an acyl group having up to 6 carbon atoms, or $R^1$ and $R^2$ together form a ring, A stands for a single bond, an oxygen atom, an NH group or a carbonyl group and n has the value 1/2 or 1, characterised in that

a) an isomeric mixture corresponding to the general formulae II-Z and II-E

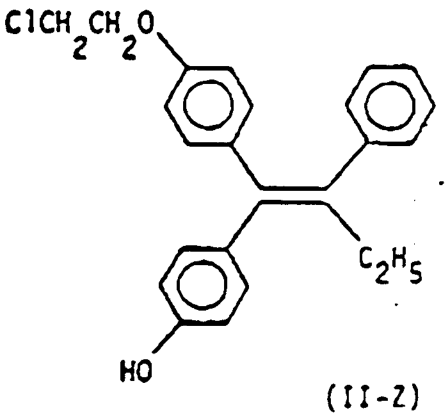

(II-Z)

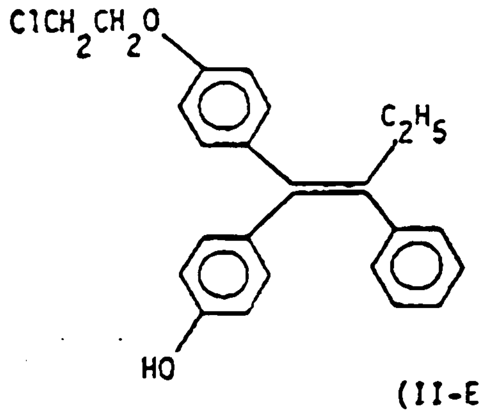

(II-E)

is reacted with an acid chloride corresponding to the general formula III

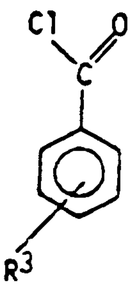

(III)

in which $R^3$ stands for a hydrogen atom, a $C_1$-$C_4$ alkyl group, an aryl group, a nitro group or a halogen atom, to form an isomeric mixture corresponding to the general formulae IV-Z and IV-E

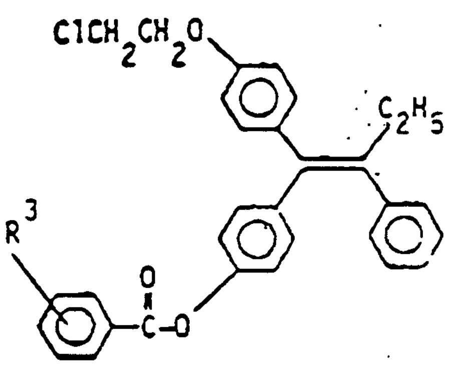

(IV-Z)

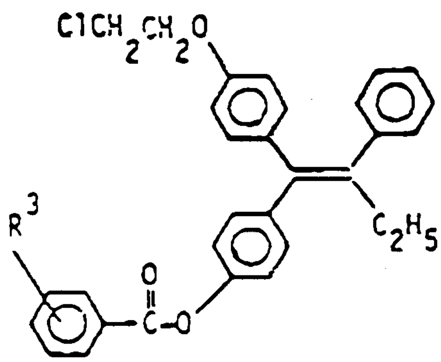

(IV-E)

b) the IV-E isomer is separated as difficultly soluble component from this isomeric mixture by fractional crystallization of compounds IV-E and IV-Z or by kinetically controlled alcoholysis or aminolysis of the compound corresponding to the general formula IV-Z, followed by filtration,
c) the resulting compound corresponding to formula IV-E is converted with dimethylamine into the compound corresponding to the general formula V-Z

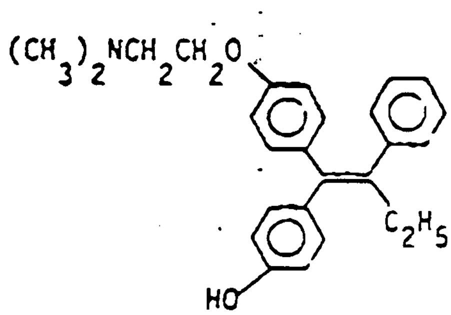

(V-Z)

d) the compound corresponding to the general formula V-Z is converted by crystallization from a chlorinated hydrocarbon (CHC) into an adduct corresponding to the general formula VI-Z

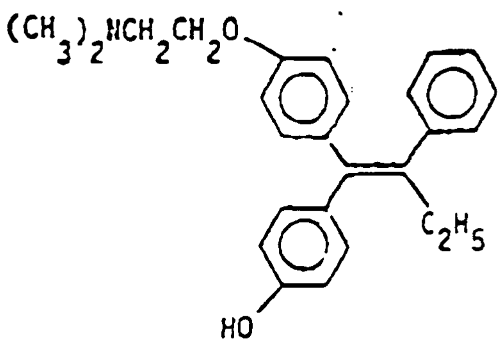

x y CHC

(VI-Z).

wherein y has the value 1/2 to 1,
and in that
e) a compound corresponding to the general formula VI-Z is converted into the stereochemichally pure and stable solvent adduct corresponding to the general formula I by crystallisation from a carbonyl compound corresponding to the general formula VII

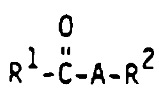

(VII)

wherein $R^1$, A and $R^2$ have the meanings indicated above.

**6.** Pharamceutical preparation, characterised in that it contains a stable solvent adduct according to claim 1 and conventional carriers and auxiliary substances.

**7.** The use of stable solvent adducts of Z-1-(p-$\beta$-dimethylaminoethoxyphenyl)-1-(p-hydroxyphenyl)-2-phenylbut-1-ene corresponding to the general formula I

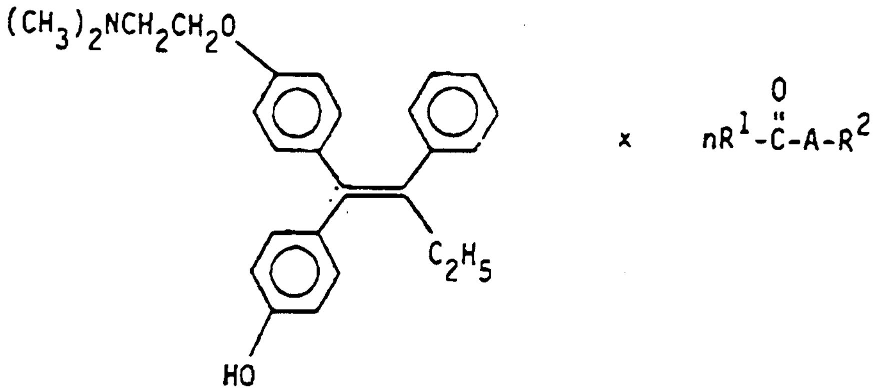

( I )

in which $R^1$ stands for a hydrogen atom, a phenyl group or a $C_1$-$C_6$ alkyl group, $R^2$ stands for a straight chain or branched $C_1$-$C_1$ alkyl group, a straight chain or branched alkenyl group or an acyl group having up to 6 carbon atoms, or R1 and R2 together form a ring, A denotes a single bond, an oxygen atom, an NH group or a carbonyl group and n has the value 1/2 or 1, for the production of a pharmaceutical preparation for the percutaneous treatment of benign and malignant tumours.

**Claim for the following Contracting States : ES, GR**

**1.** A process for the preparation of stable solvent adducts of Z-1-(p-β-dimethylaminoethoxyphenyl)-1-(p-hydroxyphenyl)-2-phenylbut-1-ene corresponding to the general formula I

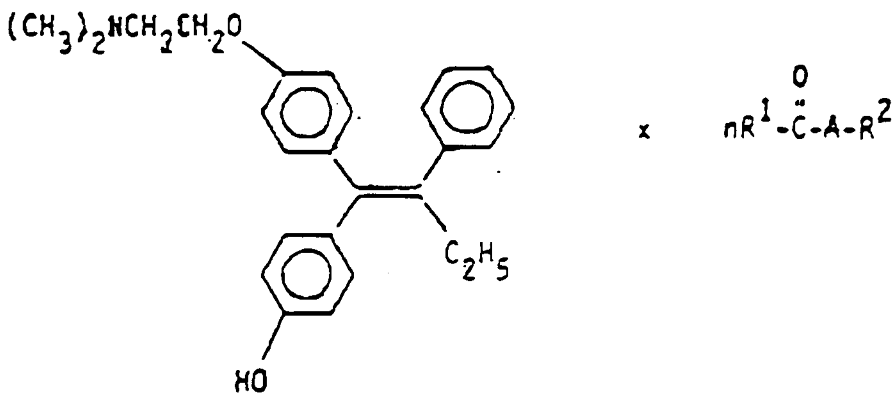

(I)

in which $R^1$ stands for a hydrogen atom, an aryl group or a $C_1$-$C_6$ alkyl group, $R^2$ stands for a straight chain or branched $C_1$-$C_6$ alkyl group, a straight chain or branched alkenyl group or an acyl group having up to 6 carbon atoms or $R^1$ and $R^2$ together form a ring, A stands for a single bond, an oxygen atom, and NH group or a carbonyl group and n has the value 1/2 or 1, characterised in that

a) an isomeric mixture corresponding to the general formulae II-Z and II-E

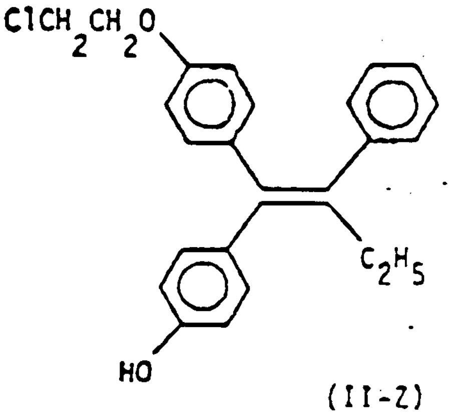

(II-Z)

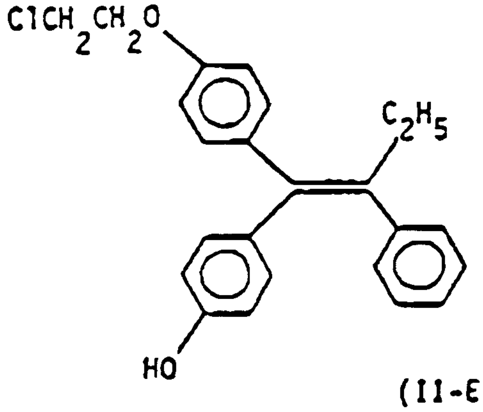

(II-E)

is reacted with an acid chloride corresponding to the general formula III

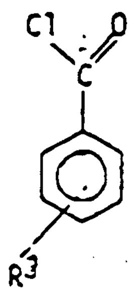

(III)

in which $R^3$ stands for a hydrogen atom, a $C_1$-$C_4$ alkyl group, an aryl group, a nitro group or a halogen atom,
to form an isomeric mixture corresponding to the general formula IV-Z and IV-E

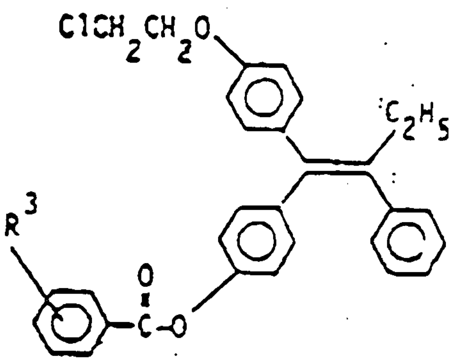

(IV-Z)

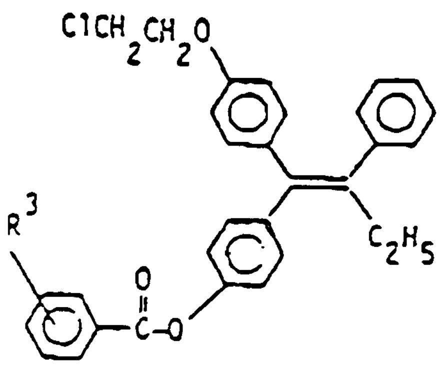

(IV-E)

b) the IV-E isomer is separated from this isomeric mixture as difficultly soluble component by fractional crystallisation of compounds IV-E and IV-Z or by kinetically controlled alcoholysis or aminolysis of the compound corresponding to the general formula IV-Z,
followed by filtration,
c) the resulting compound corresponding to the general formula IV-E is converted with dimethylamine into the compound corresponding to the general formula V-Z

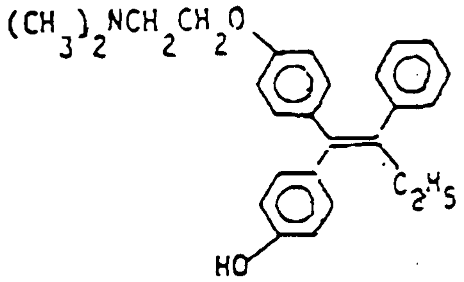

(V-Z)

d) the compound corresponding to the general formula V-Z is converted into an adduct corresponding to the general formula VI-Z by crystallisation from a chlorinated hydrocarbon (CHC).

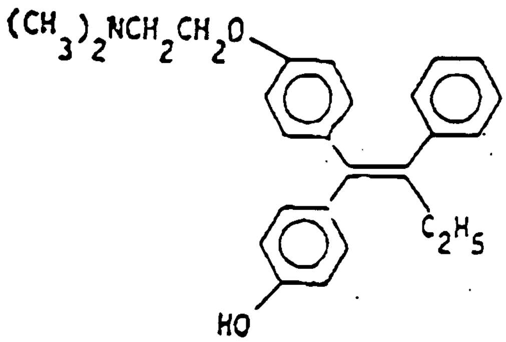 x y· CHC

(VI-Z)

wherein y has the value 1/2 to 1,
and in that
e) the compound corresponding to the general formula VI-Z is converted into the stereochemically pure and stable solvent adduct corresponding to the general formula I by crystallisation from a carbonyl compound corresponding to the general formula VII

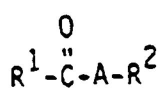

wherein $R^1$, A and $R^2$ have the meanings indicated above.

**Revendications**
**Revendications pour les Etats contractants suivants : AT, BE, CH, DE, FR, GB, IT, LI, LU, NL, SE**

**1.** Produits d'addition stables d'un solvant et de Z-1-(p-β-diméthylaminoéthoxyphényl)-1-(p-hydroxyphényl)-2-phénylbut-1-ène de formule générale I

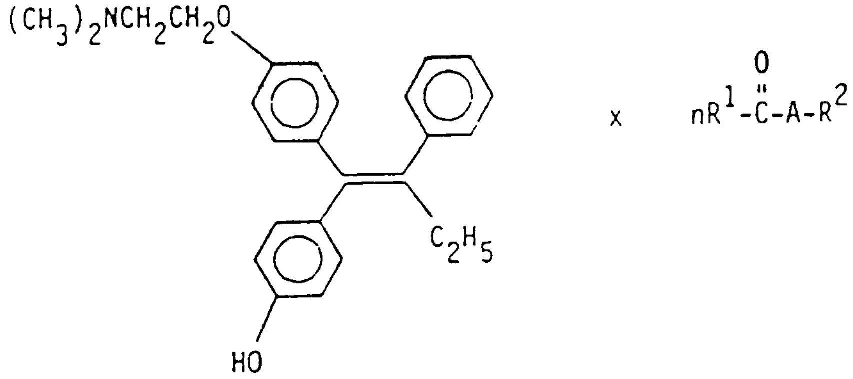

(I)

dans laquelle $R^1$ représente un atome d'hydrogène, un groupement phényle ou un groupement alkyle en $C_1$-$C_6$, $R^2$ un groupement alkyle en $C_1$-$C_6$ linéaire ou ramifié, un groupement alcényle linéaire ou ramifié ou un groupement acyle ayant un maximum de 6 atomes de carbone ou $R^1$ et $R^2$ forment ensemble un noyau, A désigne une liaison simple, un atome d'oxygène, un groupement NH- ou un groupement carbonyle et n a la valeur de 1/2 ou 1.

2. Produits d'addition selon la revendication 1, caractérisés en ce que $R^1$ et $R^2$ désignent chacun un groupement méthyle, que A représente une liaison simple et que n a la valeur de 1.

3. Produits d'addition selon la revendication 1, caractérisés en ce que $R^1$ représente un groupement méthyle, $R^2$ désigne le groupement $-CH=C(CH_3)_2$, A représente une liaison simple et n a la valeur de 1/2.

4. Produits d'addition selon la revendication 1, caractérisés en ce que le solvant est de l'acétone, de l'acétylacétone ou de l'oxyde de mésityle.

5. Procédé de préparation de produits d'addition stables d'un solvant et de Z-1-(p-$\beta$-diméthylaminoéthoxyphényl)-1-(p-hydroxyphényl)-2-phénylbut-1-ène de formule générale I

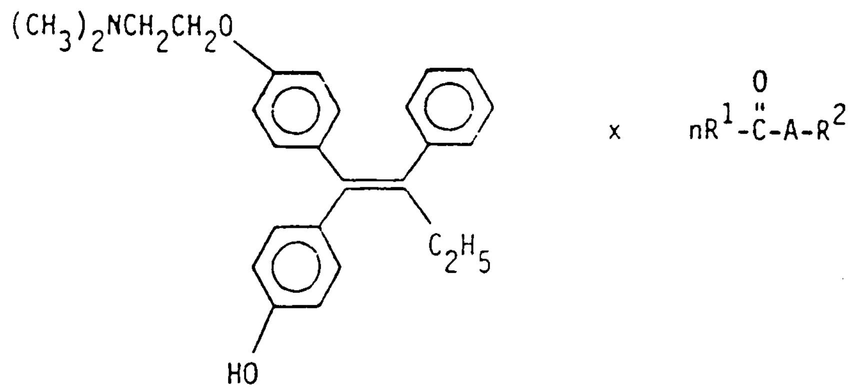

(I)

dans laquelle $R^1$ représente un atome d'hydrogène, un groupement aryle ou un groupement alkyle en $C_1$-$C_6$, $R^2$ un groupement alkyle en $C_1$-$C_6$ linéaire ou ramifié, un groupement alcényle linéaire ou ramifié ou un groupement acyle ayant un maximum de 6 atomes de carbone ou $R^1$ et $R^2$ forment ensemble un noyau, A désigne une liaison simple, un atome d'oxygène, un groupement NH- ou un groupement carbonyle et n a la valeur de 1/2 ou 1, caractérisé en ce qu'on

a) met à réagir un mélange d'isomères des formules générales II-Z et II-E

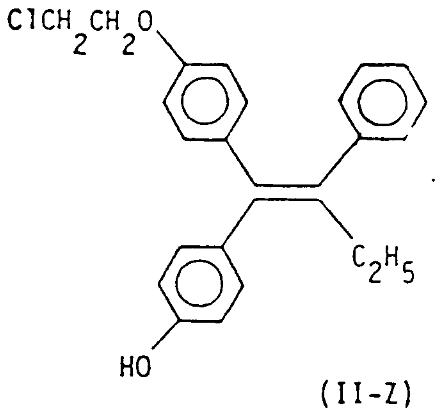

(II-Z)

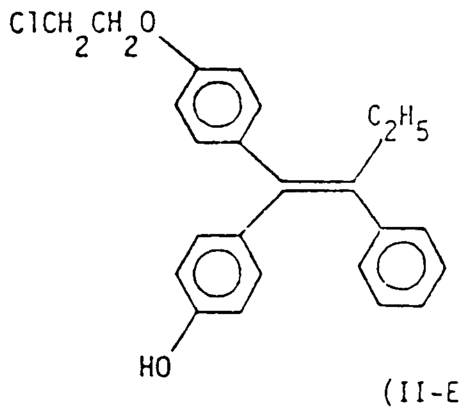

(II-E)

avec un chlorure d'acide de formule générale III

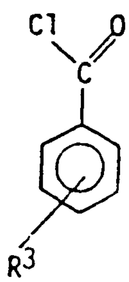

(III)

dans laquelle $R^3$ représente un atome d'hydrogène, un groupement alkyle en $C_1$-$C_4$, un groupement aryle, un groupement nitro ou un atome d'halogène, pour former un mélange d'isomères des formules générales IV-Z et IV-E

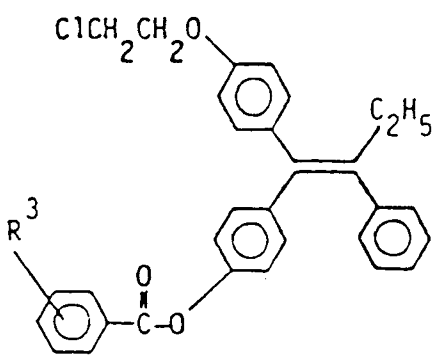

(IV-Z)

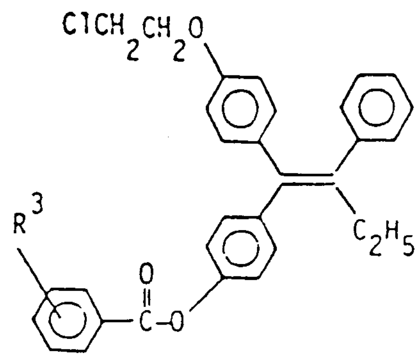

(IV-E)

b) sépare de ce mélange d'isomères, par cristallisation fractionnée des composés IV-E et IV-Z ou par alcoolyse ou aminolyse contrôlée cinétiquement du composé de formule générale IV-Z l'isomère IV-E en tant que partie plus difficilement soluble, par filtration,

c) convertit le composé ainsi obtenu de formule générale IV-E avec la diméthylamine en un composé de formule générale V-Z

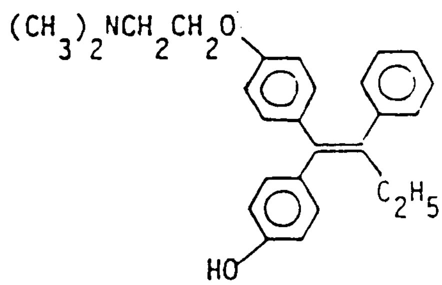

(V-Z)

d) convertit le composé de formule générale V-Z par cristallisation à partir d'un hydrocarbure chloré (CKW) en un produit d'addition de formule générale VI-Z

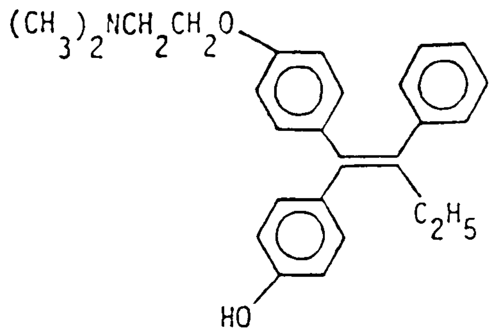

x yCKW

(VI-Z)

,

dans laquelle y a la valeur de 1/2 à 1,
et qu'on
e) transforme le composé de formule générale VI-Z par cristallisation à partir d'un groupement carbonyle de formule générale VII

$$R^1-\overset{\overset{\displaystyle O}{\|}}{C}-A-R^2 \qquad (VII)$$

dans laquelle $R^1$, A et $R^2$ possèdent les significations indiquées ci-dessus, en produit d'addition de solvant stéréochimiquement pur et stable de formule générale I.

**6.** Médicament, caractérisé en ce qu'il contient un produit d'addition stable de solvant selon la revendication 1 ainsi que des véhicules et adjuvants usuels.

**7.** Usage de produits d'addition stables d'un solvant et de Z-1-(p-$\beta$-diméthylaminoéthoxyphényl)-1-(p-hydroxyphényl)-2-phénylbut-1-ène de formule générale I

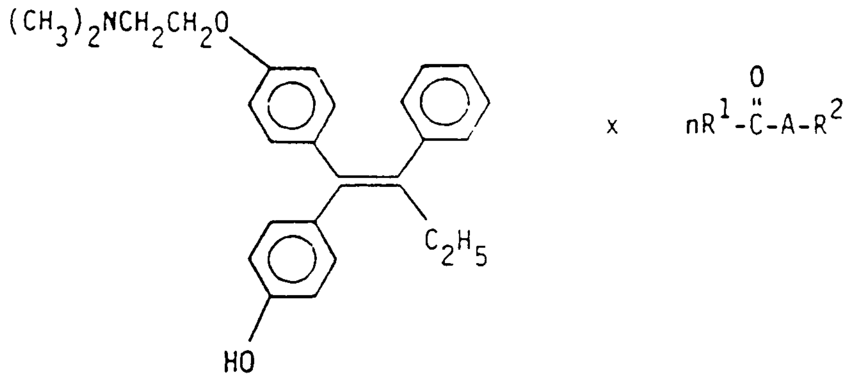

(I)

dans laquelle $R^1$ représente un atome d'hydrogène, un groupement phényle ou un groupement alkyle en $C_1$-$C_6$, $R^2$ un groupement alkyle en $C_1$-$C_6$ linéaire ou ramifié, un groupement alcényle linéaire ou ramifié ou un groupement acyle ayant un maximum de 6 atomes de carbone ou $R^1$ et $R^2$ forment ensemble un noyau, A désigne une liaison simple, un atome d'oxygène, un groupement NH- ou un groupement carbonyle et n a la valeur de 1/2 ou 1, en vue de la préparation d'un médicament destiné au traitement percutané de tumeurs bénignes et malignes.

**Revendication pour les Etats contractants suivants : ES, GR**

**1.** Procédé de préparation de produits d'addition stables d'un solvant et de Z-1-(p-β-diméthylaminoéthoxyphényl)-1-(p-hydroxyphényl)-2-phénylbut-1-ène de formule générale I

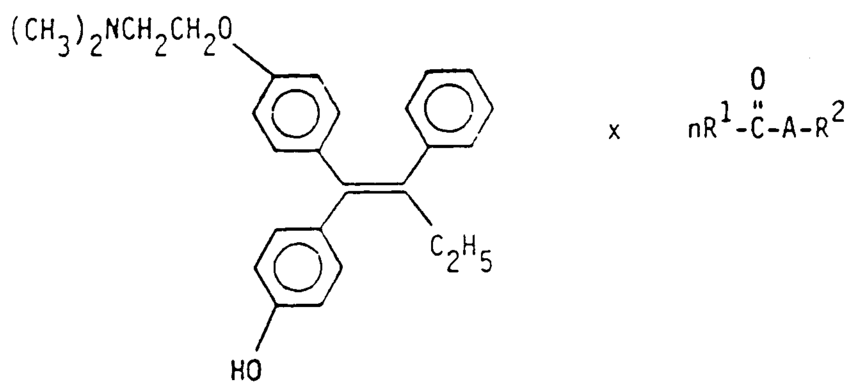

(I)

dans laquelle $R^1$ représente un atome d'hydrogène, un groupement aryle ou un groupement alkyle en $C_1$-$C_6$, $R^2$ un groupement alkyle en $C_1$-$C_6$ linéaire ou ramifié, un groupement alcényle linéaire ou ramifié ou un groupement acyle ayant un maximum de 6 atomes de carbone ou $R^1$ et $R^2$ forment ensemble un noyau, A désigne une liaison simple, un atome d'oxygène, un groupement NH- ou un groupement carbonyle et n a la valeur de 1/2 ou 1, caractérisé en ce qu'on

a) met à réagir un mélange d'isomères des formules II-Z et II-E

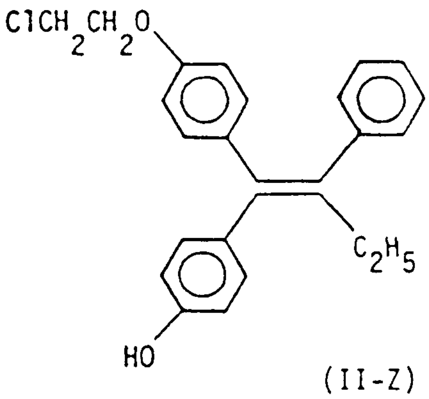

(II-Z)

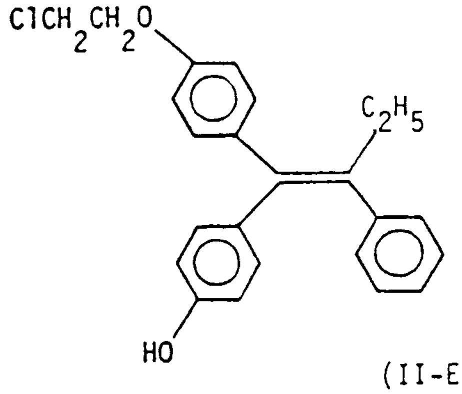

(II-E)

avec un chlorure d'acide de formule générale III

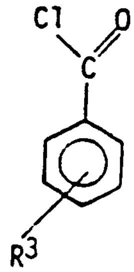

(III)

dans laquelle $R^3$ représente un atome d'hydrogène, un groupement alkyle en $C_1$-$C_4$, un groupement aryle, un groupement nitro ou un atome d'halogène, pour former un mélange d'isomères des formules générales IV-Z et IV-E

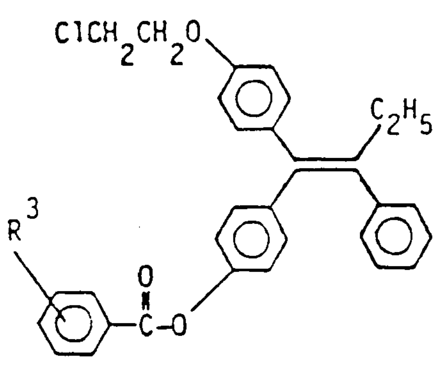

(IV-Z)

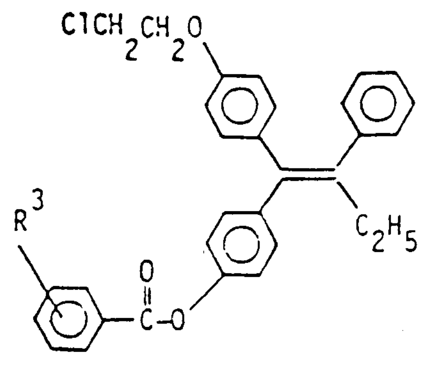

(IV-E)

b) sépare de ce mélange d'isomères, par cristallisation fractionnée des composés IV-E et IV-Z ou par alcoolyse ou aminolyse cinétiquement contrôlée du composé de formule générale IV-Z l'isomère IV-E en tant que partie plus difficilement soluble, par filtration,

c) convertit le composé ainsi obtenu de formule générale IV-E avec la diméthylamine en composé de formule générale V-Z

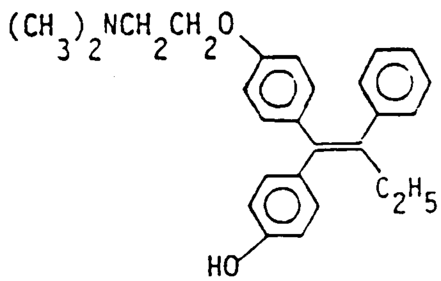

(V-Z)

d) convertit le composé de formule générale V-Z par cristallisation à partir d'un hydrocarbure chloré (CKW) en un produit d'addition de formule générale VI-Z

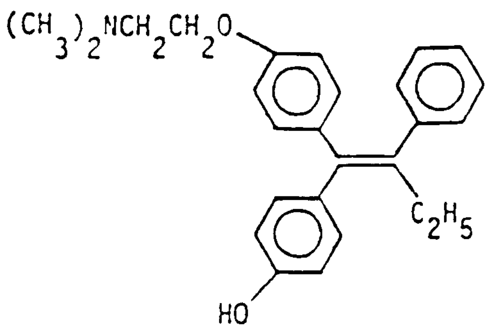

x yCKW

(VI-Z)

dans laquelle y a la valeur de 1/2 à 1,
et qu'on
e) transforme le composé de formule générale VI-Z par cristallisation à partir d'un composé carbonyle de formule générale VII

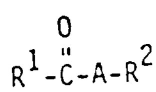

(VII)

dans laquelle $R^1$, A et $R^2$ possèdent les significations indiquées ci-dessus, en produit d'addition de solvant stéréochimiquement pur et stable de formule générale I.